# EUROPÄISCHE PATENTSCHRIFT

(11) **EP 3 324 860 B1**
(45) Veröffentlichungstag und Bekanntmachung des Hinweises auf die Patenterteilung: **07.10.2020**
(21) Anmeldenummer: 16735873.8
(22) Anmeldetag: 06.07.2016
(51) Int. Cl.: A61B 17/16, A61B 17/84, B25B 15/04, A61B 17/00

(54) **PLATZSPARENDE RATSCHEINHEIT MIT FREILAUF**
SPACE-SAVING RATCHET WITH FREEWHEEL
CLIQUET À ROUE LIBRE À ENCOMBREMENT RÉDUIT

(30) Priorität: 22.07.2015 DE 102015111878
(43) Veröffentlichungstag der Anmeldung: 30.05.2018
(73) Patentinhaber: Aesculap AG, 78532 Tuttlingen (DE)
(72) Erfinder: STEINHAUSER, Jan, 72488 Sigmaringen (DE); PFISTER, Ralf, 78647 Trossingen (DE)
(74) Vertreter: Winter, Brandl, Fürniss, Hübner, Röss, Kaiser, Polte - Partnerschaft mbB
(86) Internationale Anmeldenummer: PCT/EP2016/065986
(87) Internationale Veröffentlichungsnummer: WO 2017/012876

(56) Entgegenhaltungen:
- DE-A1- 19 942 292
- DE-A1-102011 088 252

## Beschreibung

### Hintergrund der Erfindung

Die Erfindung betrifft eine Ratsche für ein/eines medizinisches Gerät, wie eine Bohr-, Fräs- und/oder Schraubmaschine zum Bohren/Fräsen in einen Knochen und/oder Setzen von Schrauben in den Knochen oder zum Anschrauben eines Implantats, mit einem Drehmomenteinleitorgan und einem Drehmomentausleitorgan, zwischen denen eine schaltbare Verzahnungseinrichtung so angeordnet ist, dass in Abhängigkeit von einer Schaltstellung der Verzahnungseinrichtung Drehmoment vom Drehmomenteinleitorgan zum Drehmomentausleitorgan in nur eine auswählbare Drehrichtung weitergegeben wird.

### Stand der Technik

Benachbarter Stand der Technik ist bspw. aus der DE 10 2011 088 252 A1 bekannt. Dort wird ein Ratschaufsatz zum Antrieb von Schraubwerkzeugen offenbart, die zum Lösen und Festziehen von Schraubelementen dienen und die wahlweise in beiden Drehrichtungen antreibbar sind, wobei der Ratschaufsatz sowohl als geradliniger Aufsatz als auch als Winkelaufsatz ausführbar ist. In einem hülsenförmigen Gehäuse sind zwei im Abstand voneinander angeordnete, relativ zu dem Gehäuse drehbar aber axial feststehend gelagerte Mitnehmer vorgesehen. Die Mitnehmer weisen an ihren einander zugewandten Stirnseiten in ihrem Aufwandsbereich je eine Ratschverzahnung auf, die gegenläufig zueinander ausgebildet sind. Gegenüber der jeweiligen Ratschverzahnung der beiden Mitnehmer ist ein Antriebsring vorgesehen, der auf der dem jeweiligen Mitnehmer zugewandten Seite mit einer entsprechenden, mit diesem koppelbaren, Ratschverzahnung versehen ist. Der Antriebsring ist in dem Gehäuse relativ zu diesem axialverschiebbar gelagert und mittels Federeinrichtungen gegen den jeweiligen Mitnehmer abgestützt. Ferner gehört zu dem Ratschaufsatz ein Antriebswerkzeug, welches drehbar durch eine Vielecköffnung des Mitnehmers hindurchführbar und mit seinem Kupplungsende drehfest in ein Vieleckprofil des Antriebsrings einrastbar ist. Der Antriebsring bzw. der jeweilige Antriebsring ist mit Hilfe des Antriebswerkzeugs axial von demjenigen Mitnehmer, durch welchen das Antriebswerkzeug hindurchführbar ist, in einer von diesem entkoppelte Stellung wegschiebbar und gleichzeitig in eine gekoppelte Stellung mit dem anderen Mitnehmer, der das Schraubwerkzeug trägt, verschiebbar.

Dieser Ratschaufsatz ist für konventionelle Steckschlüssel vorgesehen. Zwei drehbar gelagerte, axial unverschiebbare Mitnehmer mit Sägeverzahnungen sind enthalten. Ein nicht drehbarer, axial verschiebbarer Antriebsring mit beidseitiger Verzahnung wird eingesetzt. Durch das Verschieben des Antriebsrings kann zwischen einem Links- und Rechtslauf gewechselt werden.

Zum Stand der Technik gehört ein Ringratschenschlüssel (DE 20 2011 100 987 U1) oder ein Maul-Ringratschenschlüssel, desen Wirkrichtung mittels eines Hebels umschaltbar ist, sodass beispielsweise Muttern sowohl festgezogen als auch gelöst werden können.

Eine weitere Ratscheinrichtung ist auch aus der DE 297 23 472 U1 bekannt. Darin ist eine Ratscheinrichtung zum Betätigen einer Stecknuss, z.B. einer Sechskantstecknuss, offenbart, mit einem Antriebsteil mit Vorkehrungen zur Krafteinleitung, einem konzentrisch zum Antriebsteil angeordneten Abtriebsteil mit einem an seiner äußeren Stirnseite zentrisch herausragenden Vierkantzapfen zum Eingreifen in die Vierkantöffnung einer Stecknuss, und einem Ratschen- bzw. Mitnehmerelement zwischen dem Antriebs- und dem Abtriebsteil. Als Besonders ist dabei herausgestellt worden, dass in Drehachsrichtung aufeinanderfolgend zwei Körperteile mit wesentlichen gleicher Ausbildung seiner Außenmantelfläche vorgesehen sind, wobei zumindest das Antriebsteil in seiner dem Abtriebszapfen axial entgegengesetzte Stirnseite eine Vierkantstecköffnung aufweist.

Eine Schraubendreher-Ratscheinrichtung, die umschaltbar ist, wird in Weiterentwicklung des letztgenannten Standes der Technik in der DE 199 51 888 A1 offenbart. Die Druckschrift offenbart einen umschaltbaren Ratschen-Schraubendreher, der zwar innerhalb eines Gehäuses axial hintereinander angeordnete, gegenläufige Freiläufe enthält, einen zentrischen Werkzeugschaft und in Höhe der Freiläufe einen Mitnehmerabschnitt besitzt, der mit den Freiläufen wahlweise in Aktivstellung links / rechts bringbar ist. Als Wesentlich ist herausgestellt worden, dass der Schraubendreher nach Art eines Kugelschreibers aufgebaut ist, mit einem schlanken, rohrförmigen Gehäuse, einem aus dem Gehäuse vorne herausstehenden Schaft und am hinteren Ende herausstehenden, gegen eine Federkraft wirkenden Schalt-Druckknopf, wobei am Gehäuse zudem ein Befestigungs-Clip vorgesehen ist.

Allerdings sind hier, wie auch in der vorgenannten Gebrauchsmusterschrift zwei Freiläufe mit innenliegender, entgegengesetzter Verzahnung eingesetzt. Eine Schiebewelle mit außenliegender Verzahnung wird ergänzend verwendet. Durch eine axiale Bewegung der Schiebewelle kann zwischen einem Links- und einem Rechtslauf gewechselt werden.

Bei den bestehenden Lösungen gibt es jedoch eine Vielzahl von Mängeln, die noch abgestellt werden müssen. So ziehen die Lösungen einen hohen axialen Platzbedarf nach sich, und haben meist auch einen hohen radialen Platzbedarf. Es ist kein komfortabler Wechsel des Bedienmodus möglich. Meist muss der Ratschaufsatz umgedreht werden. Die bestehenden Lösungen sind auch nicht effizient mit zusätzlichen Schalteinrichtungen kombinierbar, etwa mit einem Freilauf. Selbst wenn ein Freilauf eingesetzt ist, ist dies nur durch Herausschieben der Verschiebewelle möglich. Ein Wiedereinführen von dieser ist aber zumeist hakelig. Außerdem ist in diesem Falle durch die Anordnung der Freiläufe die Reihenfolge der durchfahrenen Schaltpositionen definiert.

Es ist die Aufgabe der vorliegenden Erfindung, die Nachteile aus dem Stand der Technik abzustellen oder wenigstens zu mildern. Vorzugsweise soll die Umsetzung einer schaltbaren Ratscheinheit in einem zylindrischen Gehäuse bei geringem axialem (und ggf. radialem) Platzbedarf vorgestellt werden. Zusätzlich soll die Möglichkeit vorgehalten werden, einen Freilauf zu schalten, ohne die Richtungseinstellung zu ändern. Besonders gewünscht ist eine Richtungseinstellung von Links- auf Rechtslauf, auch unter Einbeziehung einer direkt verbunden Schaltstellung, in dieser oder anderen Reihenfolge. Auch die Zwischenschaltung des Direktverbundenseins zwischen dem Links- und Rechtslauf von der einen oder anderen Seite her kommend, ist also wünschenswert.

Diese Aufgabe wird durch eine Ratsche mit den Merkmalen des Anspruchs 1 gelöst. Vorteilhafte Weiterbildungen der Erfindung sind Gegenstand der Unteransprüche.

Der Kern der Erfindung besteht demzufolge darin, dass die Verzahnungseinrichtung zwei zwiebelschalenartig angeordnete Drehmomentweitergabehülsen (axial ineinander geschobene Drehmomentweitergabehülsen) aufweist, die so angeordnet und beschaffen sind, dass beide mit einer (axial davon beabstandeten) Zwischenhülse, etwa nach Art einer Ausrastwelle, in vorzugsweise stirnseitigem, drehmomentweitergebendem Kontakt (Zahn-Kämmeingriff) bringbar sind. Die Drehmomentweitergabehülsen können auch als Drehmomentweitergabehohlzylinder, Drehmomentweitergabebuchsen oder Drehmomentweitergaberinge bezeichnet werden. Auch die Zwischenhülse könnte als Zwischenhohlzylinder, Zwischenbuchse oder Zwischenring bezeichnet werden. Werden nunmehr die beiden Drehmomentweitergabehülsen axialverschoben kann die eine oder andere Drehmomentweitergabehülse wahlweise mit der Zwischenhülse in Drehmoment-Übertragungseingriff gebracht werden. Sind dabei die beiden Drehmomentweitergabehülsen mit Eingriffszähnen ausgestattet die jeweils in unterschiedliche Drehrichtungen wirken und in die entgegengesetze Drehrichtungen durchrutschen erhält man eine Ratschenfunktion mit Drehrichtungsauswahl.

In anderen Worten ausgedrückt besteht der Kern der vorliegenden Erfindung in der Bereitstellung einer Ratsche für ein medizinisches Gerät (Ratsche ist im/am medizinischen Gerät verbaut), mit dem vorstehend genannten Drehmomenteinleitorgan und dem Drehmomentausleitorgan, zwischen denen eine schaltbare Verzahnungseinrichtung (Ratschgetriebe) so angeordnet ist, dass in Abhängigkeit von einer Schaltstellung der Verzahnungseinrichtung Drehmoment vom Drehmomenteinleitorgan zum Drehmomentausleitorgan weitergegeben wird. Erfindungsgemäß weist die Verzahnungseinrichtung die zwei (zwiebelschalenartig) ineinander gelagerte Drehmomentweitergabehülsen aufweist, die so angeordnet und beschaffen sind, dass beide mit einer (axial davon beabstandeten) Zwischenhülse in drehmomentweitergebenden Kontakt verbringbar sind.

Im Konkreten wird also eine Ratsche eines medizinischen Geräts vorgeschlagen mit einem Drehmoment-Einleitorgan (z.B. Ratschgehäuse), das relativ drehbar um/bezüglich eines Drehmoment-Ausleitorgans (z.B. Abtriebswelle, die vorzugsweise im Gehäuse gelagert ist) angeordnet und mit diesem über eine, einen Drehrichtungs-schaltbaren Freilauf bildende, Verzahnungseinrichtung drehmoment-gekoppelt ist, die eine erste dreh- sowie axialfest in/mit dem Drehmoment-Einleitorgan gekoppelte Drehmoment-Weitergabehülse und eine zweite drehfest sowie axialbeweglich in/mit dem Drehmoment-Einleitorgan gekoppelte Drehmoment-Weitergabehülse hat, die in entgegengesetzte Richtungen wirkende Verzahnungen (Verzahnungseinrichtungen) aufweisen, und die in Abhängigkeit einer manuell einstellbaren Axialposition der zweiten Drehmoment-Weitergabehülse wahlweise, jedoch wechselweise mit einer axial beweglich angeordneten, sowie in Richtung hin zu den Verzahnungen der Drehmoment-Weitergabehülsen vorgespannten Zwischenhülse in (direkten/indirekten) Kämmeingriff bringbar sind, welche mit dem Drehmoment-Ausleitorgan drehmomentgekoppelt ist.

Vorzugsweise ist die Ratsche, dadurch gekennzeichnet, dass die erste Drehmoment-Weitergabehülse und die zweite Drehmoment-Weitergabehülse radial übereinanderliegend angeordnet sind.

Weiter vorzugsweise ist die Ratsche gekennzeichnet durch ein Bedienelement oder Schieber, mittels welchem die manuell einstellbare Axialposition der zweiten Drehmoment-Weitergabehülse in eine erste Position schaltbar ist, in der die zweite Drehmoment-Weitergabehülse außer Eingriff mit der Zwischenhülse ist und die Zwischenhülse durch die Vorspannung in Eingriff mit der ersten Drehmoment-Weitergabehülse steht, und in eine zweite Position schaltbar ist, in der die zweite Drehmoment-Weitergabehülse in Eingriff mit der Zwischenhülse ist und diese entgegen der Vorspannung außer Eingriff mit der ersten Drehmoment-Weitergabehülse verschiebt.

Weiter vorzugsweise ist die Ratsche dadurch gekennzeichnet, dass eine der beiden Drehmomentweitergabehülsen nach Art einer ersten Rastwelle ausgebildet ist und die andere Drehmomentweitergabehülse nach Art einer zweiten Rastwelle ausgebildet ist und/oder die Zwischenhülse axialbeweglich relativ zu einer Führungswelle angeordnet ist und diese Führungswelle axialbeweglich relativ zu dem Drehmomentausleitorgan angeordnet ist, um den Freilauf zu realisieren.

Weiter vorzugsweise ist die Ratsche dadurch gekennzeichnet, dass das Drehmomenteinleitorgan als Gehäuse ausgebildet ist, in dem die beiden Drehmomenthülsen eingehaust sind und das Drehmoment-Ausleitorgan vorzugsweise eine Abtriebswelle bildet, welche vom Gehäuse umgriffen ist.

Weiter vorzugsweise ist die Ratsche dadurch gekennzeichnet, dass die eine Drehmomentweitergabehülse und die andere Drehmomentweitergabehülse ihre jeweilige Verzahnung axial ausgerichtet an einer jeweiligen Stirnfläche aufweist.

Weiter vorzugsweise ist die Ratsche dadurch gekennzeichnet, dass die Verzahnungen (an den Drehmoment-Weitergabehülsen) jeweils als Sägeverzahnung ausgebildet sind.

Weiter vorzugsweise ist die Ratsche dadurch gekennzeichnet, dass die Verzahnungen unterschiedlich zueinander (ausgerichtet) sind.

Weiter vorzugsweise ist die Ratsche dadurch gekennzeichnet, dass die Verzahnung der ersten Rastwelle radial innerhalb oder außerhalb der Verzahnung der zweiten Rastwelle vorhanden ist.

Des Weiteren wir ein Medizinisches Gerät vorgeschlagen mit einer Ratsche mit den Merkmalen nach einem der Ansprüche 1 bis 10.

Durch die vorstehenden ggf. optionalen Merkmale lassen sich die folgenden Effekte erzielen:
So ist es von Vorteil, wenn eine der beiden Drehmomentweitergabehülsen nach Art einer ersten Rastwelle ausgebildet ist und die andere Drehmomentweitergabehülse nach Art einer zweiten Rastwelle ausgebildet ist. Der radiale Platzbedarf wird dadurch verringert.

Es ist auch von Vorteil wenn die Zwischenhülse axialbeweglich relativ zu einer Führungswelle angeordnet ist und die Führungswelle axialbeweglich relativ zu dem Drehmomentausleitorgan angeordnet ist, um einen Freilauf zu realisieren. Ein lange währender verschleißfreier Betrieb wird dadurch ermöglicht.

Um eine gute Schaltbarkeit vorzuhalten, ist es von Vorteil, wenn die erste Rastwelle drehfest und axialfest am Drehmomenteinleitorgan angebracht ist.

Dafür ist es auch zuträglich, wenn die zweite Rastwelle drehfest, aber axial verschieblich am Drehmomenteinleitorgan angebracht ist.

Um die Montage und den Aufbau zu vereinfachen, ist es von Vorteil, wenn das Drehmomenteinleitorgan als Gehäuse ausgebildet ist, insbesondere als zylindrisches Gehäuse, in dem die beiden Drehmomenthülsen, vorzugsweise vollständig, eingehaust / beherbergt sind.

Wenn die eine Drehmomentweitergabehülse und/oder die andere Drehmomentweitergabehülse eine Verzahnung an einer Stirnfläche aufweist, so kann bei nur kurzen axialen Schaltwegen eine wirkungsvolle Drehmomentweitergabe zwischen den Einzelbauteilen erreicht werden.

Um ein Durchrutschen in die eine Richtung, aber eine Drehmomentweitergabe in die andere Richtung zu ermöglichen, ist es von Vorteil, wenn die Verzahnung als Sägeverzahnung ausgebildet ist. Das Prinzip einer Ratsche, also des Drehmomentweitergebens in die eine Richtung und das Durchrutschen in die andere Richtung, etwa nach Art einer Überholkupplung oder eines Freilaufes, kann dadurch wirkungsvoll nachgebildet werden.

Ein vorteilhaftes Ausführungsbeispiel ist auch dadurch gekennzeichnet, dass die Verzahnungen unterschiedlich zueinander ausgebildet sind, vorzugsweise entgegengesetzt und/oder gegensinnig ausgestaltet sind. Es ist zweckmäßig, wenn die Verzahnung der einen Drehmomentweitergabehülse und/oder der anderen Drehmomentweitergabehülse ringartig ausgeprägt / angeordnet / ausgeformt ist.

Um ein selektives Schalten zu ermöglichen, ist es von Vorteil, wenn die Zwischenhülse axial verschieblich angeordnet ist.

Damit das Drehmoment effizient übergeben werden kann, ist es von Vorteil, wenn die Zwischenhülse drehfest mit dem Drehmomentausleitorgan verbunden ist, etwa unter Zwischenschaltung eines oder mehrerer Zwischenteile.

Ein guter Wirkungsgrad bzw. ein sicheres Funktionieren bei Rechts- und Linkslauf, sowie bei Festverbundenheit in beide Richtungen, ist gewährleistet, wenn die Zwischenhülse an einer Stirnfläche eine zur Aufnahme der Verzahnung beider Drehmomentweitergabehülsen bemessene und vorbereitete Gegenverzahnung besitzt / aufweist.

Dabei ist es vorteilhaft, wenn die Gegenverzahnung als Geradverzahnung ausgebildet ist. Natürlich sind auch klauenkupplungsartige Ausprägungen möglich.

Ein vorteilhaftes Ausführungsbeispiel ist auch dadurch gekennzeichnet, dass die zweite Rastwelle mit einem bspw. stiftartigen Bedienelement fest verbunden ist, das durch das Drehmomenteinleitorgan hindurch nach außen zum Bewegen der zweiten Rastwelle ragt, etwa durch das Gehäuse hindurch ein Längsloch durchdringend.

Um den Bauraum effizient zu nutzen, ist es von Vorteil, wenn die in Radialrichtung gemessene Höhe der Gegenverzahnung der Summe der beiden Verzahnungen der Drehmomentweitergabehülsen entspricht, +/- 5 % bis 10 %.

Eine Variante ist auch dadurch gekennzeichnet, dass die zweite Rastwelle durch eine Federeinrichtung in Axialrichtung federvorgespannt ist, etwa durch Abstützen der Federeinrichtung an der ersten Rastwelle und/oder dem Drehmomenteinleitorgan, wie dem Gehäuse, und der zweiten Rastwelle.

Es hat sich auch bewährt, wenn die Federeinrichtung als Druckfeder, etwa als Schraubendruckfeder ausgebildet ist.

Herkömmliche Ankoppelungsarten lassen sich realisieren, wenn das Drehmomentausleitorgan als Abtriebswelle ausgebildet ist.

Dabei ist es von Vorteil, wenn zwischen der Abtriebswelle und der Zwischenhülse ein Übertragungsglied angeordnet ist. Ein modularer Aufbau lässt sich dann einfacher verwirklichen.

Um nicht das Gewicht unnötig zu erhöhen, also eine besonders leichte Ratsche zu erhalten, ist es von Vorteil, wenn das Übertragungsglied als hohle Führungswelle ausgebildet ist. Insbesondere ein hohlzylindrischer Querschnitt bietet sich an.

Auch hier bietet sich eine zwiebelschalenartige Schachtelung an, etwa derart, dass die hohle Führungswelle von einem Rohr wenigstens abschnittsweise umgeben ist.

Hier ist es von Vorteil, wenn die Zwischenhülse auf dem Rohr drehfest angebracht ist oder integral mit ihm ausgebildet ist.

Auch ist es möglich, dass die drehfest und axialfest an dem Drehmomenteinleitorgan angebrachte Rastwelle / Drehmomentweitergabehülse, mit dem Gehäuse einteilig / einstückig ausgeformt ist. Alternativ kann die erste Rastwelle auch in das Gehäuse eingeschraubt werden. Dies vereinfacht die Fertigung und die Montage.

Es ist auch von Vorteil, wenn das Rohr durch eine Federvorrichtung axial vorgespannt ist, etwa in Richtung von der Zwischenhülse weg zur ersten Rastwelle.

Die Erfindung betrifft letztlich auch ein medizinisches Gerät mit einer Ratsche der erfindungsgemäßen Art.

Mit anderen Worten wird die Funktion im Wesentlichen durch drei axial verzahnte Wellen erzielt, die in einem Gehäuse sitzen. Eine axial verschiebbare, federgelagerte Ausrastwelle ohne richtungsabhängige Verzahnung wird auf einer Führungswelle geführt. Die Ausrastwelle kann sich im Eingriff mit zwei verschiedenen Rastwellen befinden. Eine rotatorisch am Gehäuse fixierte Rastwelle mit Sägezahnprofil ist rotatorisch nicht verschiebbar. Die zweite Rastwelle ist rotatorisch im Gehäuse fixiert, jedoch axial verschiebbar sowie federgelagert und besitzt außerdem ein entgegengesetztes Sägezahnprofil.

Beide Rastwellen sind ineinander liegend und daher axial sowie radial platzsparend ausgestaltet. Das Sägezahnprofil der verschiebbaren Rastwelle kann vor, über und hinter das Sägezahnprofil der festen Rastwelle geschoben werden. Dies kann mit drei Schaltpositionen, etwa eines Bedienelementes realisiert werden. Eine Feder kann der Rückstellung der verschiebbaren Rastwelle dienen.

Die Verzahnung der Ausrastwelle wird durch Federn in Richtung der Rastwelle gedrückt. Abhängig von der Position der verschiebbaren Rastwelle wird ein Rechts- oder Linkslauf oder eine Kraftübertragung in beide Richtungen (Direktverbundenheit) gewährleistet.

Die Führungswelle kann optional auf einer Abtriebswelle geführt werden, welche das Drehmoment auf ein Werkzeug oder ähnliches überträgt. Wenn nun die Führungswelle axial von der Rastwelle weggeschoben wird, nimmt sie die Ausrastwelle mit, sodass diese Kontakt zu den Rastwellen verliert. Dadurch entsteht ein verschleißfreier Freilauf. In diesem Freilaufmodus kann die Führungswelle bspw. motorisch angetrieben werden.

Bisher sind ineinanderliegende Verzahnungen zur Einsparung von Bauraum in diesem Bereich nicht bekannt. Eine axial federgelagerte Welle, die die eigentliche Ratschfunktion erfüllt, ist bisher ebenfalls noch nicht erfunden worden. Auch die Umsetzung eines Freilaufs ohne Anpassung der Schaltstellung (Links-, Rechtslauf fixiert) ist neu. Zudem kann die Schaltstellung mit einem Bedienelement komfortabel manipuliert werden.

Als Vorteile zeichnen sich geringer axialer Bauraum, geringer radialer Bauraum, nur drei zu fertigende Verzahnungen, eine einfache Verstellbarkeit durch ein Bedienelement, eine einfache Umsetzung eines Freilaufs, sehr geringe Bedienkräfte und eine gleichmäßige Übertragung der Kräfte durch eine Rundum-Verzahnung aus.

Eine solche Ratsche kann im distalen Bereich eines Schraubenaufsatzes verwendet werden. Durch den Freilauf ist es hier möglich, einen elektrischen Antrieb und einen manuellen Antrieb zu verbinden. Es wird also eine Verbesserung bei der Nutzung des axialen und radialen Bauraums geschaffen. Durch die vorgestellte verschachtelte Bauweise können diese Anforderungen montagefreundlich erfüllt werden. Darüber hinaus wird die Bedienung komfortabler und der Einsatz eines Freilaufs möglich.

Die Erfindung wird nachfolgend mit Hilfe einer Zeichnung näher erläutert. Dabei sind ein erstes Ausführungsbeispiel einer Ratsche und die Umsetzung in einem medizinischen Gerät wiedergegeben. Es zeigen:
- Fig. 1: einen Längsschnitt durch eine erfindungsgemäße Ratsche einer ersten Ausführungsform in einer ersten Schaltstellung (Rechtslauf),
- Fig. 2: einen Längsschnitt in Höhe der Linie II durch die Ratsche aus Fig. 1,
- Fig. 3: einen Längsschnitt durch die Ratsche des ersten Ausführungsbeispiels in einer dritten Schaltposition (Linkslauf), die nach einer zweiten Schaltposition (Erstverbundenheit) erreicht wird,
- Fig. 4: einen Längsschnitt entlang der Linie IV aus Fig. 3, und
- Fig. 5: einen Längsschnitt durch ein medizinisches Gerät, in dem die erfindungsgemäße Ratsche in einer zu den Fign. 1 bis 4 abgewandelten, zweiten Ausführungsform eingesetzt ist.

Die Figuren sind lediglich schematischer Natur und dienen nur dem Verständnis der Erfindung. Gleiche Elemente sind mit denselben Bezugszeichen versehen.

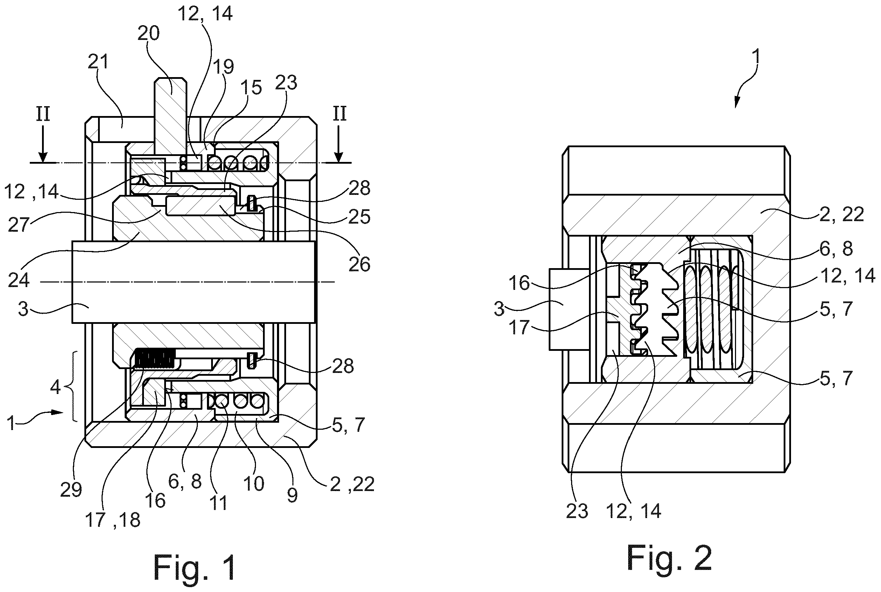

In Fig. 1 ist eine erfindungsgemäße Ratsche 1 dargestellt. Diese Ratsche 1, die auch als Ratscheneinrichtung oder Ratscheinrichtung bezeichnet werden kann, ist in einer Schaltposition 1 um einen Rechtslauf zu garantieren. Sie weist ein Drehmomenteinleitorgan (Eingangshülse/Ratschgehäuse) 2 und ein Drehmomentausleitorgan (Ausgangshülse/Abtriebswelle) 3 auf. Zwischen diesen beiden Organen 2 und 3 ist eine Verzahnungseinrichtung (Ratschgetriebe) 4 vorhanden. Die Verzahnungseinrichtung 4 ist vorzugsweise manuell schaltbar. Abhängig von einer Schaltstellung der Verzahnungseinrichtung 4 wird/kann ein Drehmoment vom Drehmomenteinleitorgan 2 auf das Drehmomentausleitorgan 3 in der entsprechend der Schaltstellung ausgewählten Drehrichtung übertragen (werden). In Abhängigkeit von der Schaltstellung und der Drehrichtung am Drehmomenteinleitorgan 2 wird demnach das Drehmoment an das Drehmomentausleitorgan 3 weitergeleitet oder nicht.

Die Verzahnungseinrichtung 4 weist zwei zwiebelschalenartig zueinander angeordnete (axial ineinander geschobene) Drehmomentweitergabehülsen 5 und 6 auf. Die eine ist also radial weiter außen bzw. innen als die andere und vorzugsweise zu dieser konzentrisch angeordnet.

Die erste Drehmomentweitergabehülse 5 ist nach Art einer ersten Rastwelle 7 ausgebildet, wohingegen die zweite Drehmomentweitergabehülse 6 nach Art einer zweiten Rastwelle 8 ausgebildet ist. Die beiden Rastwellen 7 und 8 sind vorzugsweise buchsen- oder topfartig ausgebildet und weisen in diesem bevorzugten Fall nach außen oder innen weisende Ausstülpungen auf. Eine Außenumstülpung 9 der ersten Rastwelle 7 definiert einen ringförmigen Hohlraum 10, in dem eine Federeinrichtung 11 angeordnet ist. Die Federeinrichtung 11 ist zu jedem Betriebszustand in Anlage mit der zweiten Rastwelle 8 und der ersten Rastwelle 7. Allgemein ausgedrückt deren die beiden Rastwellen 7, 8 bzw. Drehmomentweitergabehülsen 5, 6 durch die Federeinrichtung 11 axial gegeneinander (voneinander weg) vorgespannt.

Die beiden Rastwellen 7 und 8 weisen (auf der jeweils gleichen Stirnseite) Verzahnungen 12 auf. Die Verzahnung 12 der ersten Rastwelle 7 ist an einem stirnseitigen Ende der ersten Rastwelle 7 ausgebildet. Diese Verzahnung 12 ist als Sägeverzahnung 14 ausgebildet. An einem Steg 15, der von einer Außenwandung der zweiten Rastwelle 8 radial nach innen ragt, ist an der stirnseitigen Fläche des Steges 15 ebenfalls eine Verzahnung 12 ausgebildet, die ebenfalls als Sägeverzahnung 14 ausgestaltet ist. Die beiden Sägeverzahnungen 14, weisen jedoch eine gegensinnige Ausgestaltung auf. Die eine Sägeverzahnung 14 ist als Linksverzahnung ausgebildet, wohingegen die andere Sägeverzahnung 14 als Rechtsverzahnung ausgebildet ist.

Beide Sägeverzahnungen 14 können in Eingriff mit einer Gegenverzahnung 16 gebracht werden, die an einer Zwischenhülse 17 vorhanden ist, die nach Art einer Ausrastwelle 18 ausgestaltet ist. Die Ausrastwelle 18 weist auch eine Stirnfläche 19 auf, auf der die Gegenverzahnung 16 ausgebildet ist. Die Gegenverzahnung 16 ist keine Sägeverzahnung sondern eine Geradverzahnung oder Klauenkupplungsartige Verzahnung.

Ein Bedienelement 20 greift durch ein Langloch 21 in das als Gehäuse 22 ausgebildete Drehmomenteinleitorgan 2 hindurch und ist in der zweiten Drehmomentweitergabehülse 6 befestigt/in Eingriff.

Ein Rohr 23 ist drehfest und ggf. axialfest mit der Zwischenhülse 17 verbunden. Die beiden Bauteile können auch integral ausgebildet werden. Radial innerhalb der Rohres 23 ist eine Führungswelle 24 vorhanden, die eine Drehmomentweitergabe ermöglichende Außenverzahnung aufweist oder wenigstens eine Nut 25, in der ein Stift / Nutenfeder 26 eingesetzt ist. Axial ist der Stift 26 zwischen einer Schulter 27 der Führungswelle 24 und einem Sicherungsring 28 angeordnet.

Eine Federvorrichtung 29 ist eingesetzt, um eine axiale Vorspannung des Rohres 23 relativ zur Führungswelle 24 zu erzwingen.

In Fig. 2 ist die radial innenliegende Verzahnung 12 der ersten Drehmomentweitergabehülse 5, also der ersten Rastwelle 7 relativ zur zweiten Drehmomentweitergabehülse 6, also der zweiten Rastwelle 8 mit ihrer Verzahnung 12 wiedergegeben. Es ist die Sägeverzahnung 14 der ersten Rastwelle 7 in Eingriff mit der richtungsunabhängigen Gegenverzahnung 16 der Zwischenhülse 17.

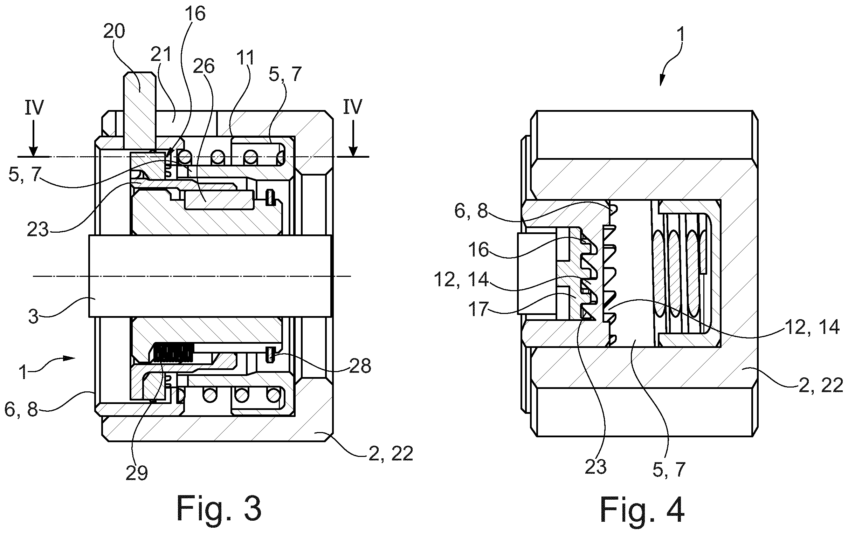

In den Fign. 3 und 4 ist eine Umschaltung in den Linkslauf dargestellt, wobei nun erkennbar ist, dass die Sägeverzahnung 14 der zweiten Rastwelle 8 in drehmomentübertragendem Eingriff mit der Gegenverzahnung 16 der Zwischenhülse 17 ist und die Zwischenhülse 17 axial verlagert ist und eben gerade nicht mehr in Eingriff mit der Sägeverzahnung 14 der ersten Drehmomentweitergabehülse 5 / ersten Rastwelle 7 steht.

Sowohl die Federeinrichtung 11, als auch die Federvorrichtung 29, wird durch eine Schraubendruckfeder ausgebildet.

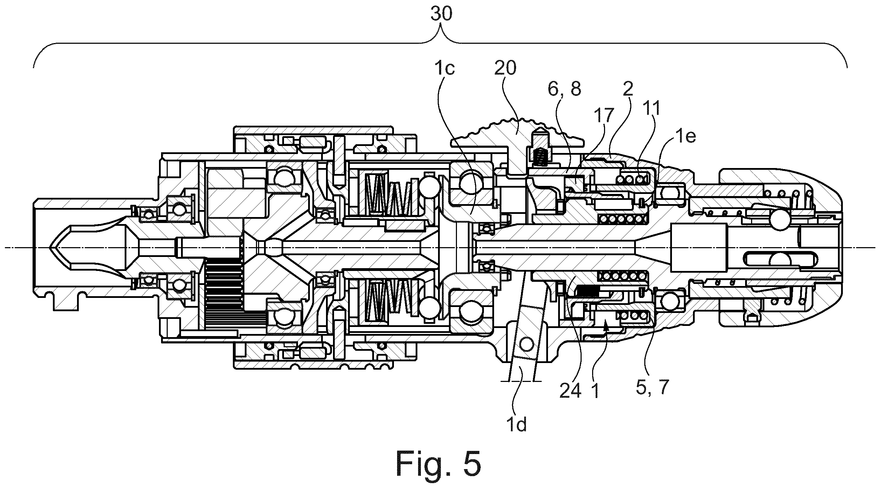

In Fig. 5 ist ein medizinisches Gerät 30 mit einer Abwandlung der Ratsche 1 wiedergegeben.

Demzufolge besteht die Ratsche 1 gemäß der Fig. 3 aus dem Gehäuse 2 (Drehmomenteinleitorgan) als eine manuelle Antriebseinheit, in welchem die zwei hülsenförmigen Drehmoment-Übertragungselemente (Drehmomentweitergabehülsen) 5, 6 drehfest in dem Gehäuse 2 aufgenommen sind, von denen zumindest ein Drehmoment-Übertragungselement 6 axialverschiebbar bezüglich des anderen Drehmoment-Übertragungselements 5 gehalten ist. Beide Drehmoment-Übertragungselemente (Drehmomentweitergabehülsen) 5, 6 weisen vorzugsweise an deren eine Stirnseite jeweils die vorstehend genannten Verzahnungen 12 auf, um wechselweise sowie wahlweise in Abhängigkeit ihrer axialen Relativlage zueinander mit der Zwischenhülse bzw. Zwischen-/Auswahlbauteil 17 vorzugsweise in Form einer axial zu den Drehmoment-Übertragungselementen benachbarten Scheibe oder eines Rings in Kämmeingriff gebracht zu werden und um so jeweils ein Drehmoment in Uhrzeiger- oder Gegenuhrzeigersinn auf die Zwischenhülse 17 zu übertragen.

Die relative Axialposition der beiden Drehmomentweitergabehülsen 5, 6 lässt sich mittels des manuell betätigbaren Schiebers/Bedienelements 20 einstellen. Der Schieber 20 ist dabei mit einem der beiden Drehmoment-Übertragungselemente 5, 6 gekoppelt, um dieses axial zu verschieben. Dadurch wird das zumindest eine axial verschiebbare Drehmoment-Übertragungselement mit dem Zwischen-/Auswahlbauteil 17 in oder außer Kämmeingriff gebracht. Das Zwischen-/Auswahlbauteil 17 ist ebenfalls axialverschieblich gehalten und mittels einer Feder in Richtung hin zu den beiden Drehmoment-Übertragungselementen 5, 6 vorgespannt. Wird somit das eine Drehmoment-Übertragungselement 6 mittels des Schiebers 20 gegen das Zwischen-/Auswahlbauteil 17 axial in Kämmeingriff gedrückt, wird dieses gegen die Federvorspannung axial weiter verschoben, wodurch der Kämmeingriff mit dem anderen Drehmoment-Übertragungselement 5 aufgehoben wird und umgekehrt.

Die Verzahnungen der beiden Drehmoment-Übertragungselemente 5, 6 wirken dabei wie vorstehend bereits ausgeführt wurde, gegenläufig zueinander, derart, dass sie nur ein Rechts- oder Links-Drehmoment übertragen können und in die jeweils andere Richtung einen Freilauf bewirken. Je nach ausgewähltem Drehmoment-Übertragungselement kann so nur ein Rechts- oder Links-wirkendes Drehmoment ausgehend von der manuellen Antriebseinheit (Gehäuse 2) über die Ratsche/ das Ratschgetriebe übertragen werden. Das Zwischen-/Auswahlbauteil 17 ist ferner axialverschieblich aber drehfest auf der Führungshülse (hülsenförmigen Ratschen-Ausgangselement 24 gelagert, das wiederum drehfest aber axialverschieblich auf der Welle der abtriebsseitigen Kupplung 3 gelagert ist.

Das Ratschen-Ausgangselement 24 hat vorzugsweise stirnseitig eine Verzahnung, über die das Ratschen-Ausgangselement 24 in Abhängigkeit seiner Axialposition bezüglich der Welle der abtriebsseitigen Kupplung 3 mit einem Ausgangselement 1c der eingangsseitigen Kupplung in drehmoment-übertragenden Kämmeingriff kommen kann, um ein Drehmoment vom Motor auf die ausgangs-/abtriebsseitige Kupplung zu übertragen.

Die manuelle Antriebseinheit (Gehäuse) 2 wird über einen Hebel 1d aktiviert und angetrieben. Dafür ist der Hebel 1d nach Art einer Kurbel ausgebildet und an die Größe einer menschlichen Hand angepasst. D.h. der Hebel 1d ist im Gehäuse 2 derart gelagert, dass das Gehäuse 2 über den Hebel 1d um die Mittelachse der Werkzeugaufnahme gedreht und damit die Drehmoment-Übertragungselemente 5, 6 angetrieben werden können. Der Hebel 1d ist aber zudem in Axialrichtung der Werkzeugaufnahme schwenkbar gelagert, wie dies in der Fig. 5 gezeigt ist. Ein in das Gehäuseinnere vorragender Hebelfortsatz ist mit dem Ratschen-Ausgangselement 24 so gekoppelt, dass dieses durch ein Verschwenken des Hebels 1d in Axialrichtung verschoben werden kann.

Das Ratschen-Ausgangselement 24 hat eine Mitnahme in Form des Radialvorsprungs oder Wellenrings 28, der direkt oder indirekt auf das Zwischen-/Auswahlbauteil 17 der Ratsche einwirkt, um dieses entsprechend der Axialbewegung des Ratschen-Ausgangselements (Führungshülse) 24 ggf. mitzunehmen.

Wird demnach das Ratschen-Ausgangselement 24 über den Hebel 1d axial gegen das Ausgangselement 1c der eingangsseitigen Kupplung in drehmoment-übertragenden Kämmeingriff verschoben, wird das Zwischen-/Auswahlbauteil 17 der Ratsche in eine Axialposition mitgenommen, in der es nicht mehr in Kämmeingriff mit einem der zwei Drehmoment-Übertragungselemente 5, 6 kommen kann. In diesem Fall wird zwar ein Drehmoment vom Motor auf die ausgangsseitige Kupplung 3 übertragen, die Ratschenfunktion wird aber außer Kraft gesetzt. Wird hingegen das Ratschen-Ausgangselement 24 über den Hebel 1d axial vom Ausgangselement 1c der eingangsseitigen Kupplung weg verschoben (kein Kämmeingriff mehr), wird das Zwischen-/Auswahlbauteil 17 der Ratsche in eine Axialposition mitgenommen/gedrückt, in der es in Kämmeingriff mit einem der zwei Drehmoment-Übertragungselemente 5, 6 kommen kann. In diesem Fall kann zwar kein Drehmoment vom Motor auf die ausgangsseitige Kupplung 3 übertragen werden, die Ratschenfunktion wird aber in Kraft gesetzt, um ein am Hebel 1d manuell eingebrachtes Drehmoment zu übertragen. Dadurch wird quasi eine Trennkupplung realisiert.

### Bezugszeichenliste

- 1: Ratsche
- 1c: Ausgangselement
- 1d: Hebel
- 2: Drehmomenteinleitorgan
- 3: Drehmomentausleitorgan
- 4: Verzahnungseinrichtung
- 5: erste Drehmomentweitergabehülse
- 6: zweite Drehmomentweitergabehülse
- 7: erste Rastwelle
- 8: zweite Rastwelle
- 9: Außenumstülpung
- 10: Hohlraum
- 11: Federeinrichtung
- 12: Verzahnung
- 14: Sägeverzahnung
- 15: Steg
- 16: Gegenverzahnung
- 17: Zwischenhülse
- 18: Ausrastwelle
- 19: Stirnfläche
- 20: Bedienelement
- 21: Langloch
- 22: Gehäuse
- 23: Rohr
- 24: Führungswelle
- 25: Nut
- 26: Stift / Bolzen
- 27: Schulter der Führungswelle
- 28: Sicherungsring
- 29: Federvorrichtung
- 30: medizinisches Gerät

## Patentansprüche

1. Ratsche (1) für medizinisches Gerät (30) mit einem Drehmoment-Einleitorgan (2) das drehbar relativ um oder bezüglich eines Drehmoment-Ausleitorgans (3) angeordnet und mit diesem über eine, einen Drehrichtungs-schaltbaren Freilauf bildende, Verzahnungseinrichtung (4) drehmoment-gekoppelt ist, die eine erste drehsowie axialfest in oder mit dem Drehmoment-Einleitorgan (2) gekoppelte Drehmoment-Weitergabehülse (5) und eine zweite drehfest sowie axialbeweglich in oder mit dem Drehmoment-Einleitorgan (2) gekoppelte Drehmoment-Weitergabehülse (6) hat, die in entgegengesetzte Richtungen wirkende Verzahnungen (12, 14) aufweisen, und die in Abhängigkeit einer manuell einstellbaren Axialposition der zweiten Drehmoment-Weitergabehülse (6) wahlweise, jedoch wechselweise mit einer axial beweglich angeordneten, sowie in Richtung hin zu den Verzahnungen (12, 14) der Drehmoment-Weitergabehülsen (5, 6) vorgespannten Zwischenhülse (17) in Kämmeingriff bringbar sind, welche mit dem Drehmoment-Ausleitorgan (3) drehmomentgekoppelt ist.

2. Ratsche nach Anspruch 1, **dadurch gekennzeichnet, dass** die erste Drehmoment-Weitergabehülse (5) und die zweite Drehmoment-Weitergabehülse (6) radial übereinanderliegend angeordnet sind.

3. Ratsche nach Anspruch 2, **gekennzeichnet durch** ein Bedienelement (20), mittels welchem die manuell einstellbare Axialposition der zweiten Drehmoment-Weitergabehülse (6) in eine erste Position schaltbar ist, in der die zweite Drehmoment-Weitergabehülse außer Eingriff mit der Zwischenhülse (17) ist und die Zwischenhülse (17) durch die Vorspannung in Eingriff mit der ersten Drehmoment-Weitergabehülse (5) steht, und in eine zweite Position schaltbar ist, in der die zweite Drehmoment-Weitergabehülse in Eingriff mit der Zwischenhülse (17) ist und diese entgegen der Vorspannung außer Eingriff mit der ersten Drehmoment-Weitergabehülse (5) verschiebt.

4. Ratsche (1) nach einem der Ansprüche 1 bis 3 , **dadurch gekennzeichnet, dass** die erste Drehmomentweitergabehülse (5) als eine erste Rastwelle (7) ausgebildet ist und die zweite Drehmomentweitergabehülse (6) als eine zweite Rastwelle (8) ausgebildet ist und/oder die Zwischenhülse (17) axialbeweglich relativ zu einer in der Ratsche angeordnete Führungswelle (24) angeordnet ist und diese Führungswelle (24) axialbeweglich relativ zu dem Drehmomentausleitorgan (3) angeordnet ist, um den Freilauf zu realisieren.

5. Ratsche (1) nach einem der Ansprüche 1 bis 4, **dadurch gekennzeichnet, dass** das Drehmomenteinleitorgan (2) als Gehäuse (22) ausgebildet ist, in dem die beiden Drehmomenthülsen (5, 6) eingehaust sind und das Drehmoment-Ausleitorgan (3) vorzugsweise eine Abtriebswelle bildet, welche vom Gehäuse (22) umgriffen ist.

6. Ratsche (1) nach einem der Ansprüche 1 bis 5, **dadurch gekennzeichnet, dass** die erste Drehmomentweitergabehülse (5) und die zweite Drehmomentweitergabehülse (6) ihre jeweilige Verzahnung (12) axial ausgerichtet an einer jeweiligen Stirnfläche aufweist.

7. Ratsche (1) nach Anspruch 6, **dadurch gekennzeichnet, dass** die Verzahnungen (12) jeweils als Sägeverzahnung (14) ausgebildet sind.

8. Ratsche (1) nach Anspruch 6 oder 7, **dadurch gekennzeichnet, dass** die Verzahnungen (12) unterschiedlich zueinander sind.

9. Ratsche (1) nach einem der Ansprüche 6 bis 8, **dadurch gekennzeichnet, dass** die Verzahnung (12) der ersten Rastwelle (7) radial innerhalb oder außerhalb der Verzahnung (12) der zweiten Rastwelle (8) vorhanden ist.

10. Medizinisches Gerät (30) mit der Ratsche (1) nach einem der vorherigen Ansprüche.

11. Medizinisches Gerät (30) nach Anspruch 10, **dadurch gekennzeichnet, dass** es eine medizinische Bohr- oder Schraubmaschine ist.

## Claims

1. A ratchet (1) for a medical device (30) comprising a torque-input element (2) which is arranged so as to be relatively rotatable around or with respect to a torque-output element (3) and, for torque transfer, is coupled to the latter via a toothed unit (4) which forms a freewheel with selectable sense of rotation and has a first torque transfer sleeve (5) which is coupled to or in the torque-input element (2) in a non-rotatable and axially fixed manner and a second torque transfer sleeve (6) which is coupled to or in the torque-input element (2) so as to be rotationally fixed therewith but axially movable, which comprise toothings (12, 14) that act in opposite directions, and which depending on a manually adjustable axial position of the second torque transfer sleeve (6) can be selectively but alternately brought into meshing engagement with an intermediate sleeve (17) which is arranged so as to be axially movable, is preloaded toward the toothings (12, 14) of the torque transfer sleeves (5, 6) and is coupled to the torque-output element (3) for torque transfer.

2. The ratchet according to claim 1, **characterized in that** the first torque transfer sleeve (5) and the second torque transfer sleeve (6) are arranged so as to be radially superimposed.

3. The ratchet according to claim 2, **characterized by** an operating element (20) by means of which the manually adjustable axial position of the second torque transfer sleeve (6) can be switched to a first position in which the second torque transfer sleeve is disengaged from the intermediate sleeve (17) and the intermediate sleeve (17) is in engagement with the first torque transfer sleeve (5) due to the preload, and to a second position in which the second torque transfer sleeve is in engagement with the intermediate sleeve (17) and shifts the latter against the preload out of engagement with the first torque transfer sleeve (5).

4. The ratchet (1) according to one of claims 1 to 3, **characterized in that** the first torque transfer sleeve (5) is formed as a first detent shaft (7) and the second torque transfer sleeve (6) is formed as a second detent shaft (8) and/or the intermediate sleeve (17) is arranged to be axially movable relative to a guide shaft (24) arranged in the ratchet, said guide shaft (24) being arranged to be axially movable relative to the torque output element (3) in order to realize the freewheel.

5. The ratchet (1) according to one of claims 1 to 4, **characterized in that** the torque-input element (2) is formed as a housing (22) in which the two torque sleeves (5, 6) are accommodated and the torque-output element (3) preferably forms an output shaft which is surrounded by the housing (22).

6. The ratchet (1) according to one of claims 1 to 5, **characterized in that** the first torque transfer sleeve (5) and the second torque transfer sleeve (6) have their respective toothing (12) provided in axial alignment on a respective end face.

7. The ratchet (1) according to claim 6, **characterized in that** the toothings (12) are formed in each case as a saw toothing (14).

8. The ratchet (1) according to claim 6 or 7, **characterized in that** the toothings (12) differ from each other.

9. The ratchet (1) according to one of claims 6 to 8, **characterized in that** the toothing (12) of the first detent shaft (7) is provided radially inside or outside the toothing (12) of the second detent shaft (8).

10. A medical device (30) comprising the ratchet (1) according to one of the preceding claims.

11. A medical device (30) according to claim 10, **characterized in that** it is a medical drilling or screwing machine.

## Revendications

1. Cliquet (1) pour appareil médical (30) avec un organe d'entrée de couple (2) agencé rotatif relativement ou par rapport à un organe de sortie de couple (3) et couplé quant au couple avec ledit organe de sortie de couple par l'intermédiaire d'un dispositif à denture (4) formant une roue libre pouvant être associée au sens de rotation et possédant une première douille de transfert de couple (5), couplée de manière fixe en rotation et fixe axialement dans ou avec l'organe d'entrée de couple (2), et une seconde douille de transfert de couple (6), couplée de manière fixe en rotation et mobile axialement dans ou avec l'organe d'entrée de couple (2), qui présentent des dentures (12, 14) agissant dans des sens opposés, et qui, en fonction d'une position axiale ajustable manuellement de la seconde douille de transfert de couple (6), peuvent être éventuellement engrenées, mais de manière alternée, avec une douille intermédiaire (17), agencée mobile axialement et précontrainte en direction des dentures (12, 14) des douilles de transfert de couple (5, 6), qui est couplée quant au couple avec l'organe de sortie de couple (3).

2. Cliquet selon la revendication 1, **caractérisé en ce que** la première douille de transfert de couple (5) et la seconde douille de transfert de couple (6) sont agencées radialement l'une au-dessus de l'autre.

3. Cliquet selon la revendication 2, **caractérisé par** un élément de commande (20) au moyen duquel la position axiale ajustable manuellement de la seconde douille de transfert de couple (6) peut être associée à une première position dans laquelle la seconde douille de transfert de couple n'est pas en prise avec la douille intermédiaire (17) et la douille intermédiaire (17) est en prise avec la première douille de transfert de couple (5) grâce à la précontrainte, et peut être associée à une seconde position dans laquelle la seconde douille de transfert de couple est en prise avec la douille intermédiaire (17) et déplace celle-ci à l'encontre de la précontrainte de manière à ce qu'elle ne soit plus en prise avec la première douille de transfert de couple (5).

4. Cliquet (1) selon l'une quelconque des revendications 1 à 3, **caractérisé en ce que** la première douille de transfert de couple (5) est réalisée sous la forme d'un premier arbre d'encliquetage (7) et la seconde douille de transfert de couple (6) est réalisée sous la forme d'un second arbre d'encliquetage (8) et/ou la douille intermédiaire (17) est agencée de manière axialement mobile par rapport à un arbre de guidage (24) agencé dans le cliquet et ledit arbre de guidage (24) est agencé de manière axialement mobile par rapport à l'organe de sortie de couple (3) afin d'obtenir la roue libre.

5. Cliquet (1) selon l'une quelconque des revendications 1 à 4, **caractérisé en ce que** l'organe d'entrée de couple (2) est réalisé sous forme de logement (22) au sein duquel sont logées les deux douilles de transfert de couple (5, 6), et l'organe de sortie de couple (3) forme de manière préférée un arbre de sortie qui est encerclé par le logement (22).

6. Cliquet (1) selon l'une quelconque des revendications 1 à 5, **caractérisé en ce que** la première douille de transfert de couple (5) et la seconde douille de transfert de couple (6) présentent leur denture (12) respective orientée de manière axiale au niveau d'une face terminale respective.

7. Cliquet (1) selon la revendication 6, **caractérisé en ce que** les dentures (12) sont réalisées respectivement sous forme de denture en dents de scie (14).

8. Cliquet (1) selon la revendication 6 ou 7, **caractérisé en ce que** les dentures (12) sont différentes les unes des autres.

9. Cliquet (1) selon l'une quelconque des revendications 6 à 8, **caractérisé en ce que** la denture (12) du premier arbre d'encliquetage (7) est présente radialement à l'intérieur ou à l'extérieur de la denture (12) du second arbre d'encliquetage (8).

10. Appareil médical (30) muni du cliquet (1) selon l'une quelconque des revendications précédentes.

11. Appareil médical (30) selon la revendication 10, **caractérisé en ce qu'**il s'agit d'une perceuse ou visseuse médicale.
